# FASCICULE DE BREVET EUROPEEN

(11) **EP 0 633 302 B1**
(45) Date de publication et mention de la délivrance du brevet: **02.09.1998**
(21) Numéro de dépôt: 94401387.9
(22) Date de dépôt: 21.06.1994
(51) Int. Cl.: C10G 35/04

(54) **Perfectionnement au procédé de traitement de produit issu du réformage catalytique**
Verbessertes Verfahren zur Behandlung eines katalytischen Reformierungsproduktes
Improved process for treatment of a catalytic reforming effluent

(30) Priorité: 08.07.1993 FR 9308413
(43) Date de publication de la demande: 11.01.1995
(73) Titulaire: INSTITUT FRANCAIS DU PETROLE, 92502 Rueil-Malmaison (FR)
(72) Inventeur: Domergue, Bruno, F-92500 Rueil Malmaison (FR); Mank, Larry, F-78630 Orgeval (FR); Fischer, Béatrice, F-92500 Rueil Malmaison (FR)

(56) Documents cités:
- EP-A- 0 002 171
- EP-A- 0 233 956
- US-A- 3 520 800
- US-A- 4 374 726

## Description

L'invention concerne un procédé pour le réformage catalytique de charges hydrocarbonées, et plus précisément pour le traitement du produit obtenu à l'issue de la réaction de réformage.

Cette réaction se déroule généralement dans au moins un réacteur de réformage en présence d'un catalyseur et dans les conditions opératoires adéquates.
Le produit sortant du dernier réacteur peut être refroidi ou subir d'autres traitements avant d'être envoyé au traitement pour la séparation du réformat et la production d'hydrogène.

L'invention concerne ce traitement.

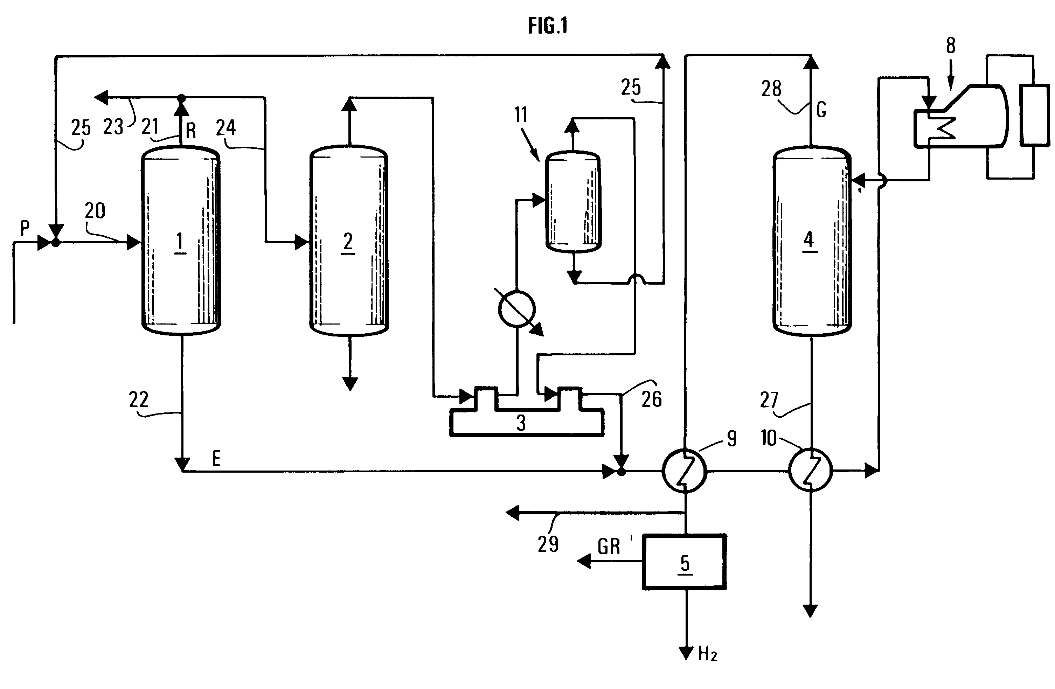

L'art antérieur connu des inventeurs est illustré par la figure 1.

Le produit P issu de la réaction de réformage (y compris ayant subi divers traitements) est introduit (canalisation 20) dans un séparateur 1 pour séparer le produit en d'une part, une phase gazeuse R riche en hydrogène (canalisation 21) et d'autre part un effluent E liquide (canalisation 22).

La phase gazeuse R à basse pression est en partie recyclée vers la réaction de réformage (canalisation 23).
L'autre partie est dirigée (canalisation 24) vers un compresseur 3, généralement à travers un ballon de garde 2.
On a représenté ici un compresseur à deux étages avec un ballon interétage 11.
La phase comprimée à au moins 10 bars obtenue est introduite dans l'effluent E (canalisation 26). La phase liquide produite lors du refroidissement intermédiaire est recyclée vers la séparation (canalisation 25).
Le mélange biphasique de l'effluent E avec la phase gazeuse comprimée est refroidi à moins de 5 °C (et en général entre -25 °C et 0 °C) avant d'être introduit dans le recontacteur (ou absorbeur) 4.

L'abaissement de la température est obtenu par échanges thermiques avec les produits sortant de l'absorbeur, c'est-à-dire avec le gaz G à travers l'échangeur 9 et avec le réformat liquide à travers l'échangeur 10. Cet abaissement de température n'étant pas suffisant, un système de réfrigération 8 doit être installé.

L'absorbeur 4 fonctionne sous pression d'au moins 10 bars et à moins de 5 °C, il permet de séparer les phases et de recueillir le réformat (canalisation 27) et le gaz G (canalisation 28) dont la pureté en hydrogène est 93-95 %.
Une partie de ce gaz G peut avantageusement être dirigée (canalisation 29) vers une unité d'hydrotraitement, en particulier de la charge du réformage.
Ce gaz passe ensuite dans un ensemble de purification 5 pour produire de l'hydrogène de grande pureté (au moins 99 et de préférence 99,9 %). Le gaz résiduel GR sortant est rejeté hors de l'installation, il est en général brûlé.

De tels procédés permettent de produire l'hydrogène très pur pour alimenter notamment l'unité d'hydrocraquage située en aval du réformage et de l'hydrogène de pureté 93 - 95 % pour l'unité d'hydrotraitement en amont.

Les inventeurs ont constaté qu'avec ces procédés seulement près de 85 % de l'hydrogène produit au réformage était récupéré.
Il est proposé dans la présente invention un procédé permettant de récupérer près de 95% (et même plus) de l'hydrogène produit théoriquement au réformage, ledit procédé étant basé sur le recyclage à pression sensiblement constante du gaz résiduel accompagnée de préférence d'une modification des conditions opératoires du recontactage.

Plus précisément, l'objet de l'invention est un procédé pour le traitement de produit issu d'une réaction de réformage catalytique de charge hydrocarbonée, comprenant :
- une séparation du produit P issu du réformage en une phase gazeuse R riche en hydrogène et un effluent E,
- un recyclage d'une partie de la phase gazeuse R obtenue à la séparation vers la réaction de réformage,
- une compression de l'autre partie de ladite phase gazeuse R à une pression d'au moins 10 bars,
- un recontactage du dit effluent E avec la phase gazeuse R comprimée, à des conditions permettant d'obtenir un gaz enrichi en hydrogène et une phase liquide enrichie en réformat,
- une purification du dit gaz G de façon à obtenir de l'hydrogène de pureté supérieure à 99 % et un gaz résiduel GR,
procédé dans lequel le gaz résiduel GR est en partie au moins recyclé dans la phase gazeuse soumise à la compression, le recyclage étant effectué à une pression sensiblement constante, et l'autre partie du gaz résiduel non recyclé étant rejetée.

De façon préférée, l'effluent E et la phase gazeuse comprimée sont recontactés à une pression comprise entre 10 et 50 bars, une température comprise entre 20 et 60 °C, pour obtenir un gaz G de pureté en hydrogène au plus égale à 95 %.

L'invention concerne également l'installation pour mettre en oeuvre ce procédé qui comprend:
- un séparateur muni d'une canalisation pour l'entrée du produit issu de réacteur de réformage, d'une canalisation pour l'évacuation d'une phase gazeuse R riche en hydrogène et d'une canalisation pour l'évacuation d'un effluent E,
- une canalisation branchée sur la canalisation d'évacuation de la phase gazeuse R du séparateur et aboutissant à une canalisation d'alimentation de réacteur de réformage, pour le recyclage d'une partie de ladite phase gazeuse R issue du séparateur,
- une boucle de compression comportant au moins un compresseur et ses utilités disposée sur la canalisation d'évacuation de la phase R pour comprimer à au moins 10 bars la phase gazeuse non recyclée issue du séparateur,
- une canalisation pour introduire ladite phase comprimée dans la canalisation amenant l'effluent E au recontacteur,
- un recontacteur muni d'une canalisation pour l'entrée de l'effluent mélangé à la phase gazeuse sous pression, une canalisation pour l'évacuation du réformat et une canalisation pour l'évacuation du gaz,
- un ensemble de purification dans lequel entre ledit gaz et duquel sort de l'hydrogène de pureté supérieure à 99 % et un gaz résiduel,
- une canalisation de recyclage du dit gaz résiduel, reliant l'ensemble de purification à la conduite alimentant la boucle de compression.
- une capacité intermédiaire située dans l'ensemble de purification ou sur la canalisation de sortie du gaz résiduel dudit ensemble.

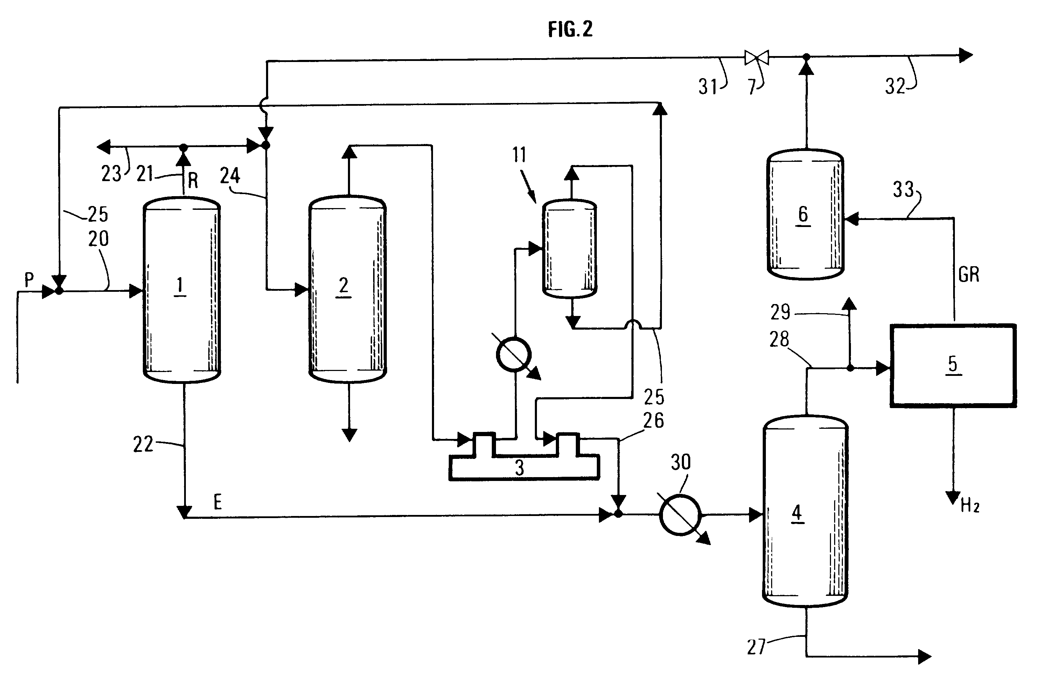

Le procédé et l'installation sont décrits à partir de la figure 2.

Le produit P contenant les hydrocarbures (essentiellement aromatiques) et l'hydrogène entre, de la même façon que dans la figure 1, dans le séparateur 1 par une canalisation 20 où il est séparé en une phase gazeuse R riche en hydrogène (canalisation 21) et un effluent E liquide (canalisation 22). Une séparation par flash est généralement utilisée.
Une partie de la phase gazeuse R à basse pression est recyclée par la canalisation 23 vers le(s) réacteur(s) de réformage.

L'autre partie de R est dirigée (canalisation 24) dans une boucle de compression comportant au moins un compresseur 3 muni de ses utilités, un ballon de garde 2 en amont du compresseur, on a représenté ici (sans que cette représentation limite l'invention) 2 étages de compression avec un ballon 11 interétage.

Une partie de la phase comprimée à au moins 10 bars, et généralement entre 10 - 50 bars, et de préférence entre 20 - 30 bars, est introduite (canalisation 26) dans la canalisation d'entrée de l'effluent dans le recontacteur 4.
De la même façon que dans l'art antérieur, le liquide issu du ballon 11 est recyclé par la canalisation 25.

Le mélange biphasique de l'effluent et de la phase comprimée est introduit dans l'absorbeur 4.
L'absorbeur (ou contacteur) 4 fonctionne de préférence entre 20 et 60 °C et sous pression d'au moins 10 bars, et généralement 10 - 50 bars, et de préférence entre 20 - 30 bars.
Un échange de chaleur classique dans un échangeur conventionnel 30 suffit alors à abaisser la température du mélange biphasique entrant dans l'échangeur à près de 70 °C.
De l'absorbeur 4 sortent le réformat contenant les aromatiques (canalisation 27) et le gaz G riche en hydrogène (canalisation 28).
Le gaz G présente ici une pureté en hydrogène d'au plus de 95 %, et en général d'environ 82 - 87 %.
La pureté en hydrogène de ce gaz G est suffisante pour l'hydrotraitement, aussi une partie de ce gaz part par la canalisation 29 vers cette unité d'hydrotraitement.

Le passage de ce gaz G dans l'ensemble de purification 5 permet d'obtenir de l'hydrogène très pur (99 % au moins, et généralement 99,9 %) et un gaz résiduel GR.

Contrairement à l'art antérieur, ce gaz résiduel n'est pas rejeté en totalité, mais une partie (50 % au moins, et plus généralement 90 - 95 % du volume gazeux) est recyclé par une canalisation 31 vers la boucle de compression, et plus précisément vers le ballon 2.

Le recyclage est effectué sous pression sensiblement constante, c'est-à-dire que la pression du gaz résiduel est écrêtée pour obtenir une variation de pression (du gaz entrant dans la boucle de compression) d'au plus ± 0,2 bars.
Un moyen pour obtenir ce résultat est de disposer une capacité intermédiaire 6 soit dans l'ensemble de purification 5 soit sur la canalisation de sortie 33 du gaz résiduel.

L'autre partie du gaz résiduel est purgée (rejetée) de façon à éviter l'accumulation d'impuretés dans la boucle de compression (canalisation 32).

Par l'utilisation du recyclage de gaz résiduel, pouvant être de plus combiné à des conditions d'absorption modifiées, et par l'emploi de compresseur de grande capacité (compresseur centrifuge), les demandeurs peuvent :
- augmenter la production d'hydrogène très pur de près de 15 %,
- éviter l'investissement d'un système de réfrigération et d'échangeurs avec les conditions de l'absorption modifiées,
- tout en maintenant une aussi bonne séparation donc une qualité similaire de réformat,
- diminuer le coût de compression grâce au recyclage qui permet d'obtenir un même taux de compression avec un nombre réduit d'étages de compression.

De tels avantages n'étaient pas envisagés par l'homme du métier qui s'était refusé jusqu'à présent à récupérer cet hydrogène résiduel devant le coût prévisible de la compression.

Pour illustrer l'intérêt de l'invention, on a comparé un procédé classique (figure 1) à l'invention (figure 2).

| | **figure 1** | **figure 2** |
|---|---|---|
| ***Conditions opératoires*** | | |
| séparateur P (bars) | 2,3 | 2,3 |
| recontacteur P (bars) | 30 | 30 |
| T (°C) | 0 | 40 |
| ensemble de purification P (bars) | 2,5 / 2,9 | 2,5 / 2,9 |
| recyclage du gaz résiduel (%) | 0 | 90 |

| ***Performances*** | | |
|---|---|---|
| * Récupération H₂ (%) | 87,5 | 97,5 |
| ** Récupération LPG (%) | 77,5 | 84,5 |
| Récupération C5+ (%) | inchangé | |

| | | |
|---|---|---|
| * Récupération H2 = quantité H2 grande pureté + quantité H2 dirigée vers l'hydrotraitement en amont / production H2 du réformage | | |
| ** Récupération LPG = quantité C3/C4 dans le réformat issu du recontacteur / production C3/C4 du réformage | | |

## Revendications

1. Procédé pour traitement de produit issu d'une réaction de réformage catalytique de charge hydrocarbonée, comprenant :
- une séparation du produit (P) issu du réformage en une phase gazeuse (R) riche en hydrogène et un effluent (E),
- un recyclage d'une partie de la phase gazeuse (R) obtenue à la séparation vers la réaction de réformage,
- une compression de l'autre partie de ladite phase gazeuse (R) à une pression d'au moins 10 bars,
- un recontactage du dit effluent (E) avec la phase gazeuse (R) comprimée, à des conditions permettant d'obtenir un gaz (G) enrichi en hydrogène et une phase liquide enrichie en réformat,
- une purification du dit gaz (G) de façon à obtenir de l'hydrogène de pureté supérieure à 99 % et un gaz résiduel (GR),
procédé caractérisé en ce que le gaz résiduel (GR) est en partie au moins recyclé dans la phase gazeuse soumise à la compression, le recyclage étant effectué à une pression sensiblement constante et l'autre partie du gaz résiduel non recyclé étant rejetée.

2. Procédé selon la revendication 1, caractérisé en ce que l'effluent (E) et la phase gazeuse comprimée sont recontactés à une pression comprise entre 10 et 50 bars, et une température comprise entre 20 et 60 °C, pour obtenir un gaz (G) de pureté en hydrogène au plus égale à 95 %.

3. Installation pour le traitement de produit issu de réacteur de réformage de charge hydrocarbonée, comprenant :
- un séparateur (1) muni d'une canalisation (20) pour l'entrée du produit issu du réacteur de réformage, d'une canalisation (21) pour l'évacuation d'une phase gazeuse riche (R) en hydrogène et d'une canalisation (22) pour l'évacuation d'un effluent (E),
- une canalisation (23) branchée sur la canalisation pour l'évacuation de la phase riche en hydrogène (R) et aboutissant à une canalisation d'alimentation de réacteur de réformage, pour le recyclage d'une partie de ladite phase gazeuse (R) issue du séparateur,
- une boucle de compression comportant au moins un compresseur (3) et ses utilités disposée sur la canalisation d'évacuation de la phase riche en hydrogène (R) pour comprimer à au moins 10 bars ladite phase gazeuse non recyclée issue du séparateur,
- une canalisation (26) pour introduire ladite phase comprimée dans la canalisation amenant l'effluent (E) au recontacteur,
- un recontacteur (4) muni d'une canalisation pour l'entrée de l'effluent mélangé à la phase gazeuse sous pression, une canalisation (27) pour l'évacuation du réformat et une canalisation (28) pour l'évacuation du gaz,
- un ensemble de purification (5) dans lequel entre ledit gaz et du quel sort par la canalisation (32) de l'hydrogène de pureté supérieure à 99 % et un gaz résiduel,
- une canalisation (31) de recyclage du dit gaz résiduel, reliant l'ensemble de purification (5) à la conduite (24) alimentant la boucle de compression,
- une capacité intermédiaire (6) située dans l'ensemble de purification (5) ou sur la canalisation de sortie du gaz résiduel dudit ensemble.

## Patentansprüche

1. Verfahren zum Verarbeiten aus einer katalytischen Reformierungsreaktion kohlenwasserstoffhaltiger Chargen stammender Produkte, umfassend
- eine Trennung des aus der Reformierung stammenden Produktes (P) in eine gasförmige an Wasserstoff reiche Phase (R) und einen Abstrom (E),
- eine Rezyklierung eines Teils der gasförmigen bei der Trennung erhaltenen Phase (R) zur Reformierungsreaktion,
- eine Kompression des anderen Teils dieser gasförmigen Phase (R) auf einen Druck von mindestens 10 bar,
- eine Rekontaktierung dieses Abstroms (E) mit der gasförmigen komprimierten Phase (R) unter Bedingungen, die es gestatten, ein an Wasserstoff reiches Gas (R) sowie eine flüssige an Reformat angereicherte Phase zu erhalten,
- eine Reinigung dieses Gases (G), derart, daß man Wasserstoff von einer Reinheit höher als 99 % sowie ein Restgas (GR) erhält,
dadurch gekennzeichnet, daß das Restgas (GR) wenigstens zum Teil in die gasförmige der Kompression ausgesetzte Phase rezykliert wird, wobei das Rezyklieren bei einem im wesentlichen konstanten Druck durchgeführt wird und der andere Teil des nicht rezyklisierten Gases verworfen wird.

2. Verfahren nach Anspruch 1, dadurch gekennzeichnet, daß der Abstrom (E) und die gasförmige komprimierte Phase bei einem Druck zwischen 10 und 50 bar und einer Temperatur zwischen 20 und 60°C rekontaktiert werden, um ein Gas (G) mit einer Wasserstoffreinheit von höchstens 95 % zu erhalten.

3. Installation für die Behandlung des aus dem Reformierungsreaktor für kohlenwasserstoffhaltige Chargen stammenden Produktes, umfassend:
- einen Separator (1), der mit einer Leitung (20) für den Eintritt des aus dem Reformierungsreaktor stammenden Produktes, einer Leitung (21) zum Abzug einer gasförmigen an Wasserstoff reichen Phase (R) und einer Leitung (22) zum Abzug eines Abstroms (E) versehen ist,
- eine Leitung (23), die an die Leitung zum Abzug der an Wasserstoff reichen Phase (R) angeschlossen ist und zu einer Speiseleitung des Reformierungsreaktors zur Rezyklierung eines Teils dieser gasförmigen aus dem Separator stammenden Phase (R) führt,
- eine Kompressionsschleife, die wenigstens einen Kompressor (3) und seine Zusatzaggregate umfaßt, angeordnet auf der Abzugsleitung für die an Wasserstoff reiche Phase (R), um diese gasförmige nicht rezyklierte aus dem Separator stammende Phase auf wenigstens 10 bar zu komprimieren,
- eine Leitung (26) zum Einführen dieser komprimierten Phase in die Leitung, die den Abstrom (E) zum Rekontaktor führt,
- einen Rekontaktor (4), der mit einer Leitung zum Eintritt des Abstroms vermischt mit der gasförmigen Phase unter Druck, einer Leitung (27) zum Abzug des Reformats und einer Leitung (28) zum Abzug des Gases versehen ist,
- eine Reinigungsanordnung (5), in der dieses Gas eintritt und aus der über die Leitung (32) Wasserstoff einer Reinheit von über 99 % sowie ein Restgas austritt,
- eine Leitung (31) zum Rezyklieren dieses Restgases, welche die Reinigungsanordnung (5) mit der Leitung (24), die die Kompressionsschleife speist, verbindet,
- einen Zwischenbunker (6), der in der Reinigungsanordnung (5) oder auf der Austrittsleitung für das Restgas dieser Anordnung angeordnet ist.

## Claims

1. A method of processing the product emanating from a catalytic reforming reaction of a hydrocarbonated feedstock comprising :
- separation of the product (P) of reforming into a hydrogen rich gaseous phase (R) and an effluent (E),
- recycling of a part of gaseous phase (R) obtained at separation and passing it to the reforming reaction,
- a compression of the other part of the said gaseous phase (R) to a pressure of at least 10 bars,
- recontacting of the said effluent (E) with the compressed gaseous phase (R) under conditions which make it possible to obtain a gas (G) which is enriched in hydrogen and a liquid phase which is enriched in reformate,
- a purification of the said gas (G) in order to obtain hydrogen of better that 99 % purity and a residual gas (GR),
the said method being characterised in that the residual gas (GR) is in part at least recycled in the gaseous phase which is subjected to compression, recycling being carried out at a substantially constant pressure, the other part of the residual gas which is not recycled being rejected.

2. A method according to claim 1 characterised in that the effluent (E) and the compressed gaseous phase are recontacted at a pressure comprised between 10 and 50 bars, and a temperature comprised between 20 and 60°C, in order to obtain a gas (G) with a hydrogen purity at least equal to 95%.

3. A plant for processing the product emanating from the reactor for reforming hydrocarbonated feedstock, comprising :
- a separator (1) provided with an inlet duct (20) for product emanating from the reforming reactor, a duct (21) for drawing off a hydrogen rich gaseous phase (R) and a duct (22) for discharging an effluent (E),
- a duct (23) connected to the duct for drawing off the hydrogen rich gaseous phase (R) from the separator and terminating at a duct for supplying the reforming reactor, for recycling a part of the said gaseous phase (R) coming from the separator,
- a compression loop comprising at least one compressor (3) and its utilities disposed on the duct for drawing off the hydrogen rich phase (R) in order to compress to at least 10 bars the gaseous phase emanating from the separator and which is not recycled,
- a duct (26) for feeding the said compressed phase into the duct carrying the effluent (E) to the recontactor,
- a recontactor (4) provided with a duct for the intake of effluent mixed with the pressurised gaseous phase, a duct (27) for drawing off the reformate and a duct (28) for drawing off the gas,
- a purification unit (5) into which the said gas enters and from which emerges through the duct (32) the hydrogen which has a purity greater than 99% and a residual gas,
- a duct (31) for recycling the said residual gas, connecting the purification unit (5) to the duct (24) supplying the compression loop,
- an intermediate container (6) situated in the purification unit (5) or on the duct through which the residual gas emerges from the said unit.
